# EUROPEAN PATENT APPLICATION

(11) **EP 1 622 058 A1**
(43) Date of publication of application: **01.02.2006**
(21) Application number: 05015910.2
(22) Date of filing: 21.07.2005
(51) Int. Cl.: G06F 19/00

(54) **Method of mapping cDNA sequences**

(30) Priority: 26.07.2004 JP 2004217652
(71) Applicant: HITACHI SOFTWARE ENGINEERING CO., LTD., Yokohama-shi, Kanagawa 230-0045 (JP)
(72) Inventor: Shishiki, Toru, Shinagawa-ku Tokyo 140-0002 (JP)
(74) Representative: Liesegang, Roland

(57) **Abstract**

A method of mapping cDNA sequences is disclosed to enable searching for matching portions between a large number of cDNA sequences and genome sequences in a short period of time. cDNA sequences sharing high homology are grouped from among a large number of cDNA sequences. A consensus sequence 701 maximally matching any of the sequences within the group is created. Matching portions between the sequence and a genome sequence 702 are searched for, and then a partial sequence 706 containing the matching portions is extracted. Matching portions between the partial sequence and cDNA sequences within the group are searched for. Accordingly, the number of instances of searching for matching portions from genome sequences is reduced so as to shorten the processing time.

SEQUENCE LISTING

<110> Hitachi Software Engineering Co., Ltd.

<120> Method of mapping cDNA sequences

<130> PH-2484

<160> 32

<170> PatentIn version 3.1

<210> 1
<211> 58
<212> DNA
<213> Artificial Sequence

<220>
<223> Synthetic DNA

<400> 1
attgccgcgc gatatttgtg ttcaacatcg cgcgtatgag tcctaatcat gctagtct 58

<210> 2
<211> 33
<212> DNA
<213> Artificial Sequence

<220>

<223>  Synthetic DNA

<400>  2
ctataaacac aagttgtacc ccgcatactc agg                                    33

<210>  3
<211>  15
<212>  DNA
<213>  Artificial Sequence

<220>
<223>  Synthetic DNA

<400>  3
aacgttgtac atact                                                        15

<210>  4
<211>  15
<212>  DNA
<213>  Artificial Sequence

<220>
<223>  Synthetic DNA

<400>  4
ttgcaacatg tatga                                                        15

<210> 5

<211> 58

<212> DNA

<213> Artificial Sequence

<220>

<223> Synthetic DNA

<400> 5

attgccgcgc gatatttgtg ttcaacatcg cgcgtatgag tcctaatcat gctagtct 58

<210> 6

<211> 18

<212> DNA

<213> Artificial Sequence

<220>

<223> Synthetic DNA

<400> 6

cgtagtgctc ctatgcgt 18

<210> 7

<211> 19

<212> DNA

<213> Artificial Sequence

<220>

<223>  Synthetic DNA

<400>  7
ttttttgggg gcccattaa                                                    19

<210>  8
<211>  18
<212>  DNA
<213>  Artificial Sequence

<220>
<223>  Synthetic DNA

<400>  8
atttttgggg gcccctaa                                                     18

<210>  9
<211>  25
<212>  DNA
<213>  Artificial Sequence

<220>
<223>  Synthetic DNA

<400>  9
ttgcgtagtg ctcctaatta tgcgt                                             25

<210> 10
<211> 19
<212> DNA
<213> Artificial Sequence

<220>
<223> Synthetic DNA

<400> 10
tttttccgg gcccattaa 19

<210> 11
<211> 19
<212> DNA
<213> Artificial Sequence

<220>
<223> Synthetic DNA

<400> 11
cgcgcgcgtt ttaaaaccc 19

<210> 12
<211> 19
<212> DNA
<213> Artificial Sequence

<220>

<223>  Synthetic DNA

<400>  12
tttcgtagtg cacctaatt                                                    19

<210>  13
<211>  10
<212>  DNA
<213>  Artificial Sequence

<220>
<223>  Synthetic DNA

<400>  13
tttaaaaacc                                                              10

<210>  14
<211>  18
<212>  DNA
<213>  Artificial Sequence

<220>
<223>  Synthetic DNA

<400>  14
cgtagtgctc ctatgcgt                                                     18

<210> 15
<211> 25
<212> DNA
<213> Artificial Sequence

<220>
<223> Synthetic DNA

<400> 15
ttgcgtagtg ctcctaatta tgcgt 25

<210> 16
<211> 19
<212> DNA
<213> Artificial Sequence

<220>
<223> Synthetic DNA

<400> 16
tttcgtagtg cacctaatt 19

<210> 17
<211> 19
<212> DNA
<213> Artificial Sequence

<220>

<223>  Synthetic DNA

<400>  17
   ttttttgggg gcccattaa                                                    19

<210>  18
<211>  18
<212>  DNA
<213>  Artificial Sequence

<220>
<223>  Synthetic DNA

<400>  18
atttttgggg gcccctaa                                                      18

<210>  19
<211>  19
<212>  DNA
<213>  Artificial Sequence

<220>
<223>  Synthetic DNA

<400>  19
ttttttccgg gcccattaa                                                     19

<210> 20

<211> 19

<212> DNA

<213> Artificial Sequence

<220>

<223> Synthetic DNA

<400> 20

cgcgcgcgtt ttaaaaccc 19

<210> 21

<211> 10

<212> DNA

<213> Artificial Sequence

<220>

<223> Synthetic DNA

<400> 21

tttaaaaacc 10

<210> 22

<211> 18

<212> DNA

<213> Artificial Sequence

<220>

<223>  Synthetic DNA

<400>  22
cgtagtgctc ctatgcgt                                                    18

<210>  23
<211>  25
<212>  DNA
<213>  Artificial Sequence

<220>
<223>  Synthetic DNA

<400>  23
ttgcgtagtg ctcctaatta tgcgt                                            25

<210>  24
<211>  19
<212>  DNA
<213>  Artificial Sequence

<220>
<223>  Synthetic DNA

<400>  24
tttcgtagtg cacctaatt                                                   19

<210> 25

<211> 24

<212> DNA

<213> Artificial Sequence

<220>

<223> Synthetic DNA

<400> 25

tkcgtagtgc tcctaattrt gcgt 24

<210> 26

<211> 27

<212> DNA

<213> Artificial Sequence

<220>

<223> Synthetic DNA

<400> 26

acctgttatt tagcgccgcg aaatgct 27

<210> 27

<211> 27

<212> DNA

<213> Artificial Sequence

<220>

<223>  Synthetic DNA

<400>  27
actgctgtca cgtactacta tctacta                                          27

<210>  28
<211>  27
<212>  DNA
<213>  Artificial Sequence

<220>
<223>  Synthetic DNA

<400>  28
tgacgtttct gctggcggga ttattat                                          27

<210>  29
<211>  22
<212>  DNA
<213>  Artificial Sequence

<220>
<223>  Synthetic DNA

<400>  29
acctgttatt tagcgccgcg aa                                               22

<210> 30

<211> 22

<212> DNA

<213> Artificial Sequence

<220>

<223> Synthetic DNA

<400> 30
acctgttatt tagcgccttg aa 22

<210> 31

<211> 22

<212> DNA

<213> Artificial Sequence

<220>

<223> Synthetic DNA

<400> 31
actacttatt tagcgccgcg aa 22

<210> 32

<211> 27

<212> DNA

<213> Artificial Sequence

<220>

<223>  Synthetic DNA

<400>  32
acctgttatt tagcgccgcg aaatgct                                          27

## Description

### CLAIM OF PRIORITY

The present applicatiob claimspriority from Japanese applicatiob JP 2004-217652 filed on July 26, 2004, the content of which is hereby incorporated by reference into this application.

### BACKGROUND OF THE INVENTION

### Field of the Invention

The present invention relates to searching (hereinafter referred to as mapping) for portions of a large genome sequence matching each of a large number of cDNA sequences.

### Description of Related Art

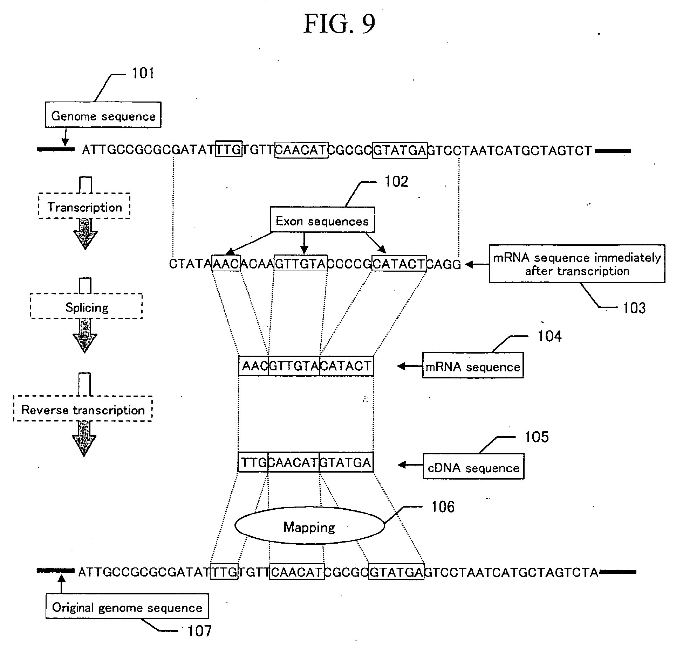

Fig. 9 illustrates the relationship between a cDNA sequence and a genome sequence and mapping of the cDNA sequence onto the genome sequence. An mRNA sequence 103 immediately after transcription from a genome sequence 101 comprises a plurality of exon sequences 102 and the other sequences. mRNA immediately after transcription is subjected to a process called splicing to produce an mRNA sequence 104 where only exon sequences are linked. When the mRNA sequence is reverse-transcribed, a cDNA sequence 105 is obtained. Mapping 106 involves searching for a portion matching the cDNA sequence from the original genome sequence 107 and locating the portion on the genome. In general, a large number of mRNA sequences are generated within cells. Thus, a large number of cDNA sequences can be obtained by reverse-transcribing the mRNA sequences. However, the full lengths of these cDNA sequences are not completely reverse-transcribed and fragments thereof are obtained. They are referred to as EST (Expressed Sequence Tag) sequences. Mapping onto genome sequences is often carried out for the EST sequences.

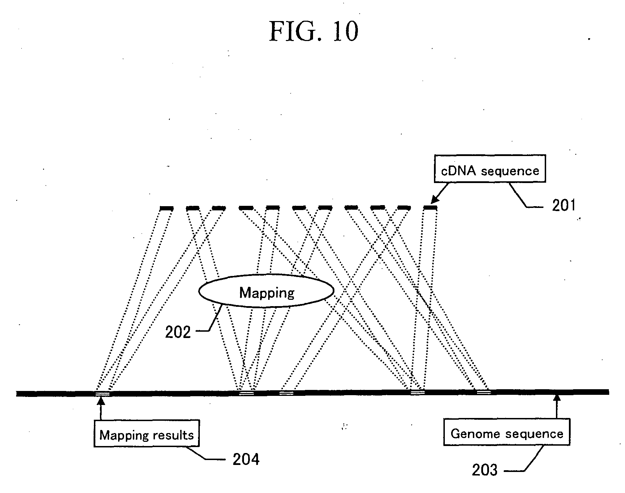

Conventionally, when a large number of cDNA sequences or EST sequences are mapped onto genome sequences, mapping positions are calculated and realized by a program on a computer. Examples of a representative program for calculating mapping positions include sim4 and Blat. Fig. 10 illustrates a conventional mapping method. A cDNA sequence 201 is mapped (202) onto a genome sequence 203, thereby obtaining mapping results 204. Such mapping results are information about to which positions of a genome sequence a plurality of exon sequences divided from a cDNA sequence to be mapped correspond. Similar processing is carried out for all the other cDNA sequences. In this way, all sequences are similarly matched one by one onto genome sequences.

[Patent document 1] JP Patent Publication (Kokai) No. 7-115959 A (1995)

### SUMMARY OF THE INVENTION

Generally, genome sequences are very large, and there are many cDNA sequences and EST sequences. Hence, there is a problem such that mapping requires a significant amount of time. For example, in the case of humans, the genome sequence length is nearly 3 billion bases and there are a hundred thousand or more EST sequences. In this case, when a 2.6 GHz dual CPU is used, mapping of all EST sequences onto the genome takes about 1 week.

The purposes of the present invention are to provide a mapping method whereby mapping can be carried out in a time shorter than that required for conventional mapping and to provide software and a system for realizing such purpose.

To achieve the above purposes, according to the present invention, clusters of cDNA sequences to be mapped onto genome sequences and sharing high homology are previously formed. Thus, the number of sequences to be mapped onto the full-lengths of large genome sequences is reduced and the processing time required for mapping a large number of cDNA sequences is shortened. The cluster information on cDNA sequences is controlled by a database.

The method of mapping cDNA sequences, and specifically, for mapping a plurality of cDNA sequences onto a genome sequence according to the present invention, uses computer for performing clustering of a plurality of sequences based on sequence-to-sequence homology, creating a consensus sequence of a plurality of sequences, and mapping one sequence onto another sequence. The computer executes the steps of: dividing a plurality ofcDNA sequences into a plurality of clusters based on sequence-to-sequence homology; creating within each cluster a consensus sequence of a plurality of cDNA sequences belonging to such cluster; mapping the consensus sequence of each cluster onto the genome sequence; extracting, for every consensus sequence, a partial sequence containing both ends of a mapping position on the genome sequence from the genome sequence as a partial sequence for mapping; and mapping each cDNA sequence within the corresponding clusters onto the relevant partial sequences for mapping.

The method of mapping cDNA sequences of the present invention can be realized by a computer program.

### BRIEF DESCRIPTION OF THE DRAWINGS

Fig. 1 shows an example of system configuration according to the present invention.
Fig. 2 is a flow chart showing the outline of the entirety of the processing according to the present invention.
Fig. 3 illustrates a method for clustering a plurality of cDNA sequences.
Fig. 4 shows procedures for creating a consensus sequence within a cluster.
Fig. 5 shows procedures for mapping a consensus sequence and extracting a partial sequence for mapping.
Fig. 6 illustrates a method for mapping a plurality of cDNA sequences within clusters.
Fig. 7 shows an example of user interface and screen flow.
Fig. 8 shows an example of a viewer for displaying mapping results.
Fig. 9 illustrates the relationship between cDNA sequences and a genome sequence and mapping of the sequences onto the genome sequence.
Fig. 10 shows a conventional mapping method.

### DETAILED DESCRIPTION OF THE PREFERRED EMBODIMENTS

An embodiment for implementing the present invention will be described specifically by referring to drawings.

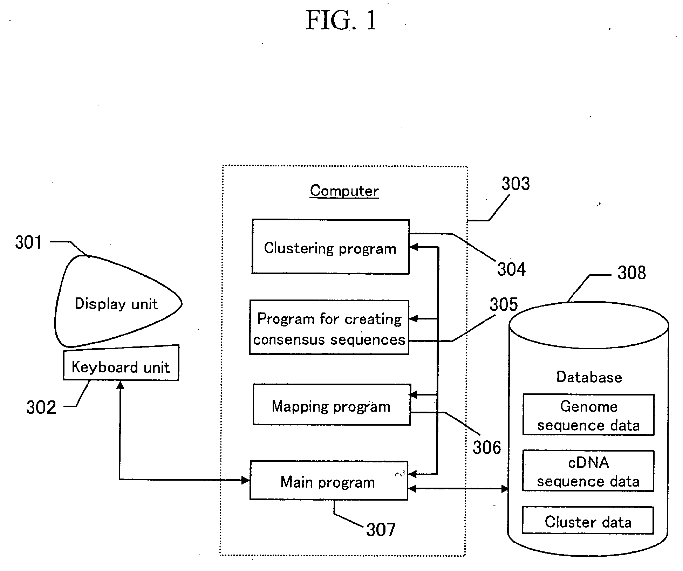

Fig. 1 shows an example of system configuration according to the present invention. A user operates a keyboard unit 302 while viewing a display unit 301 to order computer 303 to perform processing. The computer 303 has a main program 307. The main program 307 calls up a clustering program 304, a consensus-sequence-creating program 305, and a mapping program 306 to perform processing. Furthermore, the main program 307 stores results obtained by processing in a database 308 or obtains data from the database 308. In the database, genome sequence data, cDNA sequence data, and cluster data are stored.

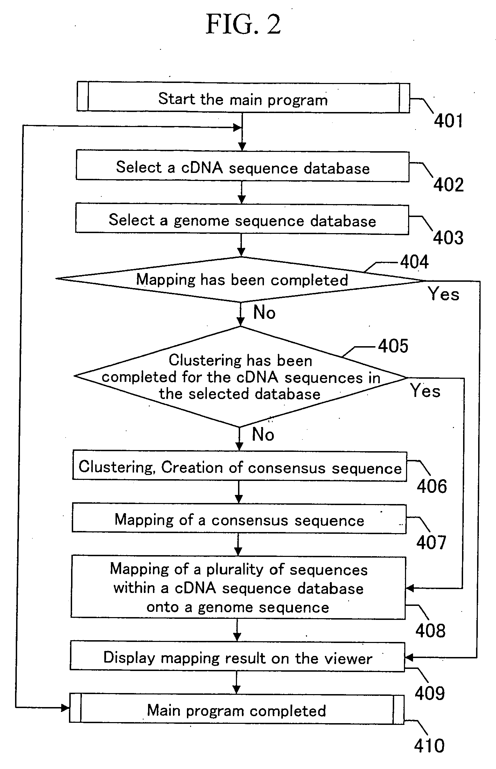

Fig. 2 is a flow chart showing the outline of the entirety of the processing according to the present invention. At the beginning, the main program is started (step 401). Next, a database of cDNA sequences to be mapped is selected (step 402). One cDNA sequence database contains, as described above, a plurality of cDNA sequences (or EST sequences). Next, a genome sequence in a database, onto which a plurality of sequences in the selected cDNA sequence database are mapped, is selected (step 403). Next, it is determined whether or not the cDNA sequences in the selected database have already been mapped onto the genome in the selected database (step 404). If mapping has been performed, the mapping result can be displayed on a viewer (step 409). If mapping has not yet been performed, it is determined whether or not clustering has already been performed for the cDNA sequences in the selected database (step 405). If clustering has been performed, mapping onto the selected genome sequence (data) is performed (step 408). If clustering has not yet been performed, clustering and creation of consensus sequences are performed (step 406) and then the consensus sequences are mapped onto the genome sequence (data) (step 407). When mapping has been completed, the mapping results can be displayed on the viewer (step 409). Finally, the main program is terminated (step 410).

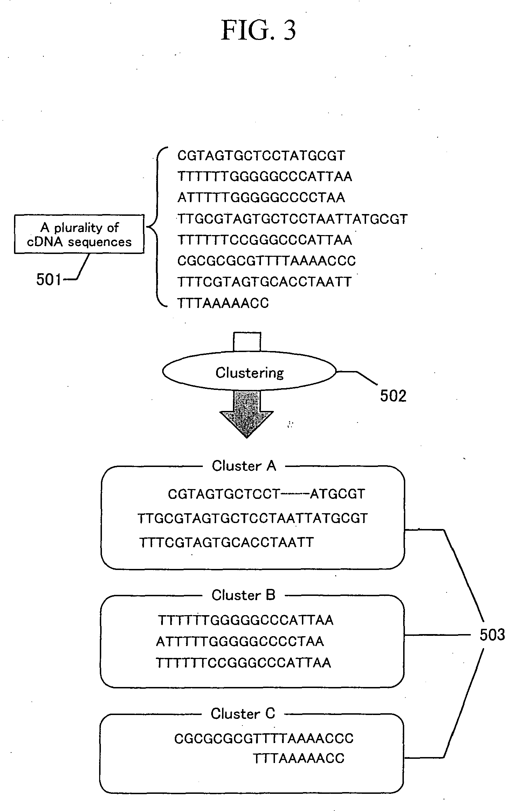

Fig. 3 illustrates clustering of cDNA sequences. Clustering is performed for cDNA sequences 501 to be mapped (502). A homology search program such as Blast, FastA, or SmithWaterman is used for clustering, so as to form a cluster 503 of cDNA sequences sharing high homology. At this time, values of parameters, which are the standards for forming clusters, can be determined. When clustering is performed, cDNA sequences reverse-transcribed from mRNA that had been transcribed from the identical gene region are ideally classified into one cluster. The number of formed clusters varies depending on the determined values of parameters. Exon regions are excised in various patterns from mRNA immediately after transcription and then linked. This is called alternative splicing. Accordingly, ideally, mRNAs generated from a gene region that is identical to that on the genome are relatively analogous to each other and are classified into one cluster.

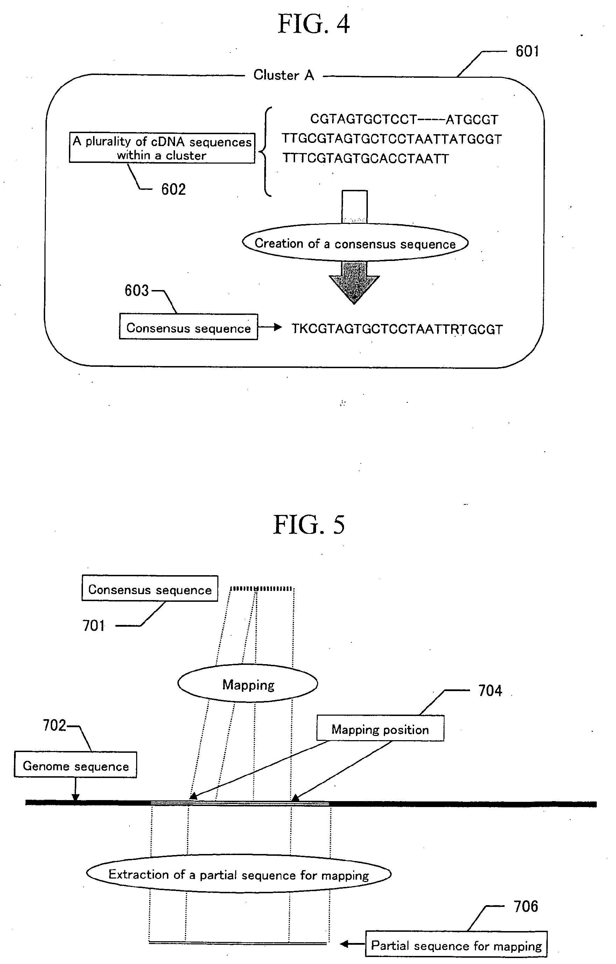

Fig. 4 shows procedures for creating a consensus sequence within a cluster. Within the identical cluster 601 among the above-formed clusters, a consensus sequence 603 is created from among cDNA sequences 602. To create a consensus sequence, a multiple alignment program such as ClustalW is used. Multiple alignment is a method that involves examining similarity among a plurality of sequences and maximizing the matching among the plurality of sequences, while placing gaps in the sequences. A consensus sequence is a sequence maximally matching any of the plurality of sequences. Only one consensus sequence is determined for a plurality of sequences.

Fig. 5 shows procedures for mapping a consensus sequence and extracting a partial sequence for mapping. The consensus sequence 701 created in Fig. 4 is mapped onto a genome sequence 702 using a mapping program such as Blat or sim4. A partial sequence containing both ends 704 of the mapping position of the consensus sequence mapped onto the genome sequence is extracted. The thus extracted sequence is determined to be a partial sequence 706 for mapping.

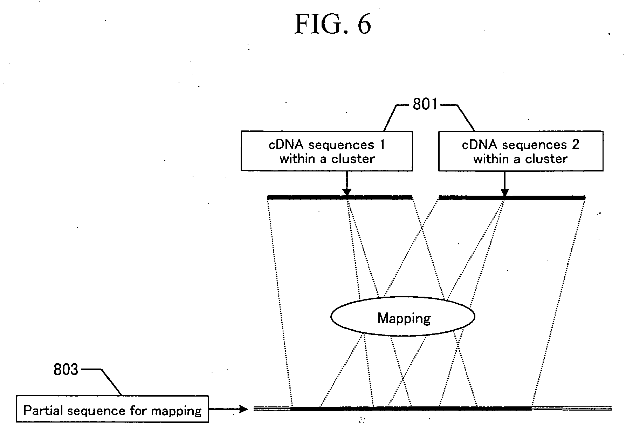

Fig. 6 illustrates a method for mapping a plurality of cDNA sequences within clusters. A plurality of cDNA sequences 801 within clusters are mapped onto the partial sequence 803 for mapping (extracted in Fig. 5). As a program to be used for mapping, a program such as blat, sim4, or blast can be selected.

By the above procedures, all cDNA sequences can be mapped onto genome sequences. According to the procedures, the number of instances of mapping onto large genome sequences is no larger than the number of clusters. Thus, time required for mapping can be shortened.

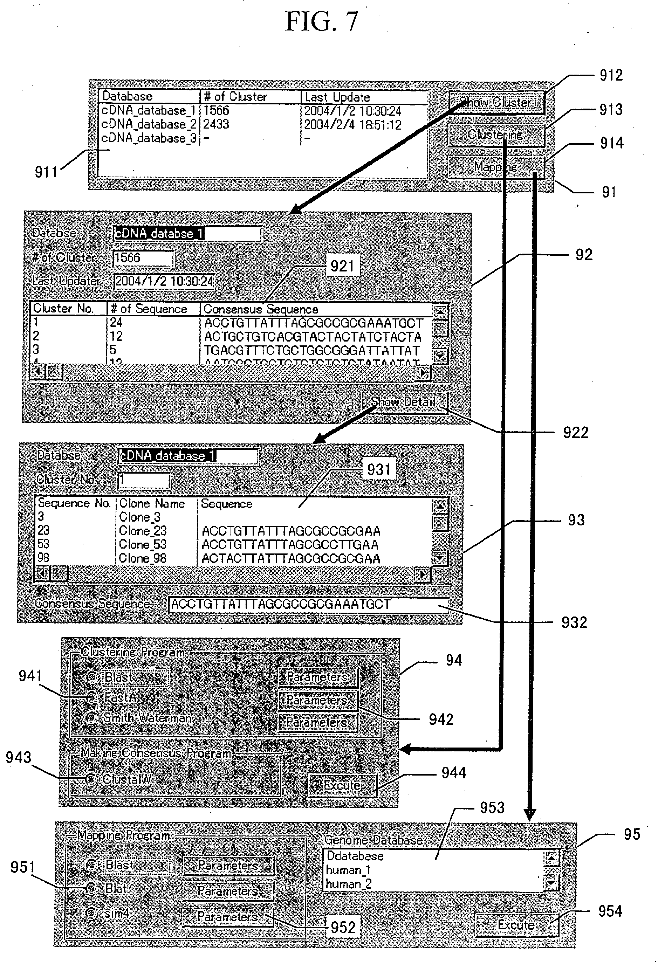

Fig. 7 shows an example of user interface and screen flow. Specifically, this is an example when cDNA sequences are mapped onto genome sequences. A dialog box 91 for displaying the list of cDNA databases displays a list 911 of cDNA sequences. In the list, "# of Cluster (the number of clusters)" and "Last Update" are displayed. "# of Cluster" displays the number of clusters that have been formed by clustering and "Last Update" displays the time at which latest clustering was performed. When clustering has not been performed, nothing is displayed. When a database of cDNA sequences that have been subjected to clustering is selected from the list, a mapping button 914 becomes available so as to enable mapping.

Furthermore, when a database cDNA of sequences that have been subjected to clustering is selected from the list and the "Show Cluster" button 912 is depressed, a dialog box 92 for displaying the list of clusters appears and then the list of clusters 921 is displayed. In the list, "Cluster No.," "# of Sequence," and "Consensus Sequence" are displayed. "Cluster No." displays serial numbers of clusters, "# of Sequence" displays the number of cDNA sequences included in clusters, and "Consensus Sequence" displays consensus sequences of clusters. When a cluster is selected from the list and then the "Show Detail" button 922 is depressed, a dialog box 93 for displaying detailed information on clusters appears, so that a list 931 of sequences within the selected cluster and the consensus sequence 932 of the cluster are displayed.

When a cDNA database is selected from the dialog box for displaying the list of cDNA databases and then the "Clustering" button 913 is depressed, a dialog box 94 for determining clustering parameters appears. A clustering program is selected (941), parameters for the selected program are determined (942), and then a program for creating a consensus sequence is selected (943). When the "Execute" button 944 is depressed, clustering is executed for sequences within the selected cDNA database. To perform mapping, the "Mapping" button 914 in the dialog box for displaying the list of cDNA databases is depressed so that a dialog box 95 for determining mapping parameters appears. A mapping program is selected (951), parameters for the program are determined (952) and then a genome in a database 953, onto which mapping is performed, is selected. When the "Execute" button 954 is depressed, sequences within the selected cDNA database are mapped onto the genome in the selected database. Meanwhile, cluster information is stored in a database, enabling mapping of cDNA sequences in the database, which have once been subjected to clustering, onto various genomes in databases in shorter time than ever before.

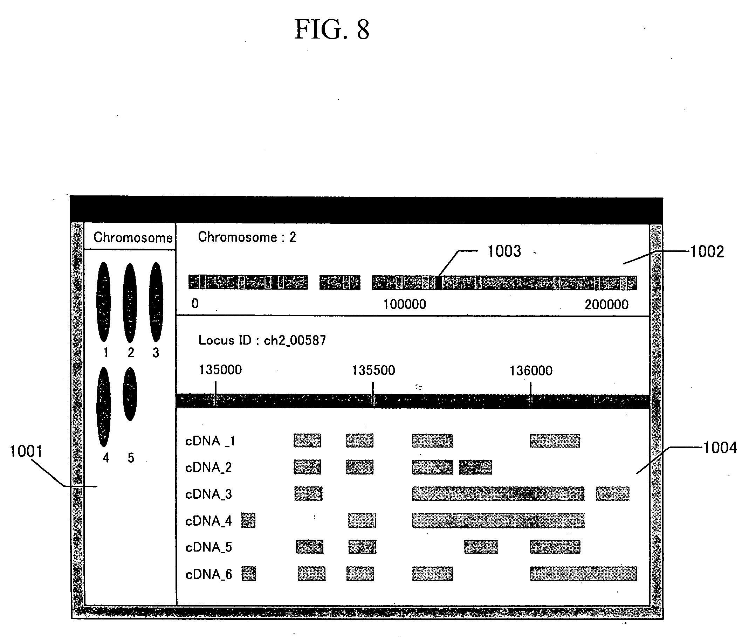

Fig. 8 shows an example of a viewer for displaying mapping results. For cDNA sequence databases for which mapping has been completed, the mapping positions can be confirmed using the viewer. When one chromosome is selected from a chromosome view 1001, the entire chromosome sequence is displayed in a genome sequence map view 1002. When one gene locus is selected from the genome sequence map view, (1003), the mapped cDNA sequences are displayed in a locus view 1004.

## Claims

1. A method of mapping a plurality of cDNA sequences onto a genome sequence, which uses a computer for performing clustering of a plurality of sequences based on sequence-to-sequence homology, creating a consensus sequence of a plurality of sequences, and mapping one sequence onto another sequence, wherein the computer executes the steps of:
dividing the plurality of cDNA sequences into a plurality of clusters based on sequence-to-sequence homology;
creating within each cluster a consensus sequence of the plurality of cDNA sequences belonging to the cluster;
mapping the consensus sequence of each cluster onto the genome sequence;
extracting, for every consensus sequence, a partial sequence containing both ends of a mapping position on the genome sequence from the genome sequence as a partial sequence for mapping; and
mapping each cDNA sequence within the corresponding clusters onto the partial sequence for mapping.

2. An apparatus for mapping a plurality of cDNA sequences onto a genome sequence, which is provided with:
a clustering unit for clustering a plurality of inputted cDNA sequences based on sequence-to sequence homology;
a consensus-sequence-creating unit for creating a consensus sequence of the plurality of cDNA sequences belonging to each cluster formed by the clustering unit; and
a mapping unit for mapping one sequence onto another sequence;
said mapping unit being provided to map the consensus sequence to each cluster created by the consensus-sequence-creating unit onto a genome sequence to extract for
every consensus sequence a partial sequence containing both ends of a mapping position on the genome sequences from the genome sequence as a partial sequence for mapping, and to map each cDNA sequence within the corresponding clusters onto the extracted partial sequence for mapping.
